# EUROPEAN PATENT APPLICATION

(11) **EP 2 450 030 A1**
(43) Date of publication of application: **09.05.2012**
(21) Application number: 10794170.0
(22) Date of filing: 30.06.2010
(51) Int. Cl.: A61K 8/37, A61K 8/19, A61K 8/63, A61Q 1/02

(54) **SOLID POWDER COSMETIC MATERIAL**

(30) Priority: 30.06.2009 JP 2009155207
(71) Applicant: Shiseido Company, Ltd., Chuo-ku Tokyo 104-8010 (JP)
(72) Inventor: SONOYAMA, Yuji, Yokohama-shi Kanagawa 224-8558 (JP); YAGI, Katsuhiko, Yokohama-shi Kanagawa 224-8558 (JP)
(74) Representative: Gendron, Vincent Christian
(86) International application number: PCT/JP2010/061113
(87) International publication number: WO 2011/002002

(57) **Abstract**

Problem: To provide a pressed powder cosmetic composition excellent in impact resistance, or a pressed powder cosmetic composition satisfying both impact resistance and good feeling in use.

Means for Resolution: A pressed powder cosmetic composition comprising oily ingredients in an amount of from 1 to 20% by mass and powder ingredients in an amount of from 80 to 99% by mass, wherein the oily ingredients include di(isostearyl/phytosteryl) dimer dilinoleate; or the pressed powder cosmetic composition which contains di (isostearyl/phytosteryl) dimer dilinoleate in a ratio of from 0.5 to 15% by mass in the total amount of the cosmetic composition.

## Description

### TECHNICAL FIELD

The present invention relates to a pressed or solidified powder cosmetic composition. In particular, the invention relates to a pressed powder cosmetic composition which contains a relatively small amount of an oily ingredient therein.

### BACKGROUND ART

A pressed powder cosmetic composition is a general purpose use cosmetic base especially in a makeup cosmetic composition, but the poor impact resistance thereof has been a serious issue. In particular, a cosmetic composition such as eye shadow or the like containing a pearlescent pigment in a high ratio therein may have a problem in its poor impact resistance due to the powdery characteristics of the pearlescent pigment. And furthermore, in view of the cosmetic product characteristics, it is more desirable for the cosmetic compositions to have a good feeling in use in addition to have a high impact resistance.

For improving the impact resistance, there is known a technique to product a pressed powder cosmetic composition by molding a specific slurry filled in a container, thereby to product the pressed powder composition, where the slurry contains a powder ingredient and a highly-viscous, solid or semisolid oily ingredient such as fluorine-containing oil agent (e.g., Patent Reference 1). However, the technique disclosed in Patent Reference 1 is problematic in that the feeling in use of the product is heavy and that the oily ingredient exerts a significant influence on the compatibility and the dispersibility of the powder therein.

On the other hand, as described in Patent Reference 2, it is known to use an ester of a dimer acid with a phytosterol and an alcohol as an oil agent for enhancing a glossiness in the oily cosmetic composition such as rouge or the like. In addition, a filling and solidifying method for a cosmetic composition is described in Patent References 3 and 4.

### PRIOR ART REFERENCES

### PATENT REFERENCES

Patent Reference 1: JP 2001-322912A
Patent Reference 2: JP 2001-199937A
Patent Reference 3: JP 57-60004B
Patent Reference 4: JP 61-54766B

### SUMMARY OF INVENTION

### PROBLEMS THAT INVENTION IS TO SOLVE

The present invention has been made in consideration of the problems with the related arts mentioned above, and one object is to provide a pressed powder cosmetic composition which is excellent in impact resistance, and another object is to provide a pressed powder cosmetic composition satisfying both high impact resistance and good feeling in use.

### MEANS FOR SOLVING THE PROBLEMS

In consideration of the above-mentioned situation, the present inventors have made efforts to study for solving the problems and, as a result, have found that, when a specific dimer acid ester is incorporated in a pressed powder cosmetic composition which contains a relatively small amount of the oily ingredient, then the cosmetic composition can have excellent impact resistance and that, when the said specific dimer acid ester is incorporated in a specific ratio amount, then the cosmetic composition can satisfy both high impact resistance and good feeling in use; and on the basis of these findings, the inventors have completed the present invention.

Specifically, the invention provides a pressed powder cosmetic composition comprising oily ingredients in an amount of from 1 to 20% by mass and powder ingredients in an amount of from 80 to 99% by mass, wherein the oily ingredients include di(isostearyl/phytosteryl) dimer dilinoleate.

The invention also provides the above-mentioned solid powder cosmetic, wherein di (isostearyl/phytosteryl) dimer dilinoleate is contained in a ratio of from 0.5 to 15% by mass in the total amount of the cosmetic composition.

### ADVANTAGEOUS EFFECTS OF INVENTION

According to the invention, a specific dimer acid ester is incorporated in a pressed powder cosmetic composition containing a relatively small amount of oily ingredients therein, to thereby enhance the impact resistance of the cosmetic composition; and further, when the specific dimer acid ester is incorporated therein in a specific ratio, then the invention attains the advantageous effects of enhancing both the impact resistance and the feeling in use of the cosmetic composition.

### MODE FOR CARRYING OUT INVENTION

The invention is described in detail hereinunder. In this description, the amount of each ingredient (% by mass) means, unless otherwise specifically indicated, the amount thereof (% by mass) based on the total amount of the pressed powder cosmetic composition as the final product.

### <Oily Ingredient>

### [Di(isostearyl/phytosteryl) Dimer Dilinoleate]

The pressed powder cosmetic composition of the invention includes di(isostearyl/phytosteryl) dimer dilinoleate as the oily ingredient therein.

Di(isostearyl/phytosteryl) dimer dilinoleate is a diester produced by bonding phytosterol and isostearyl alcohol to dimer dilinoleic acid prepared through polymerization of linoleic acid.

As the compound, herein employable are commercial products, such as "LUSPLAN PI-DA" (by Nippon Fine Chemical Co., Ltd.), etc.

The amount of di (isostearyl/phytosteryl) dimer dilinoleate is preferably from 0.5 to 15% by mass in the pressed powder cosmetic composition of the invention, more preferably from 1 to 10% by mass. When the amount is more than 15% by mass, then the adherability of the pressed powder composition to a cosmetic puff or sponge in using may be worsen, and in addition, the spreadability in applying the composition onto the skin may be poor. Even when the amount of di(isostearyl/phytosteryl) dimer dilinoleate is so small such as 0.5% by mass, it is effective for enhancing the impact resistance. In consideration of the balance of the feeling in use and the impact resistance, the most preferred amount thereof is from 1 to 10% by mass.

### [Other Oily Ingredients]

As any other oily ingredient than the above-mentioned di(isostearyl/phytosteryl) dimer dilinoleate, any one usable in cosmetic compositions can be used here, such as liquid oils and fats, solid oils, higher fatty acids, hydrocarbon oils, fluorochemical oils, organic UV absorbents, higher alcohols, ester oils, silicone oils and the like, as long as not detracting from the advantage of the invention, and they can be used either singly or in combination. These oily ingredients can function as the binder in the pressed powder cosmetic composition. Specific examples of the oily ingredients are shown below.

Exemplary liquid oils and fats include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, peanut oil, tea seed oil, nutmeg oil, rice bran oil, Chinese wood oil, Japanese wood oil, jojoba oil, germ oil, and triglycerin.

The solid oils and fats include hydrocarbon-based waxes, such as paraffin wax, Fischer-Tropsch wax, polyethylene wax (molecular weight, 300 to 2000), polyethylene/polypropylene wax (molecular weight, 300 to 2000), microcrystalline wax, and ceresin wax; natural waxes, such as carnauba wax, candelilla wax, bees wax, rice bran wax, Japanese wax, and their purified products; as well as long-chain fatty alcohols, silicone wax, cacao butter, coconut oil, horse fat, hydrogenated coconut oil, palm oil, beef tallow, mutton tallow, hydrogenated beef tallow, palm kernel oil, lard, beef bone tallow, Japanese core wax, hydrogenated oil, neatsfoot tallow, and hydrogenated castor oil.

Exemplary higher fatty acids include lauric acid, myristic acid, palmitic acid, stearic acid, behenic acid, oleic acid, undecylenic acid, tall oil acid, isostearic acid, linoleic acid, linolenic acid, eicosapentaenoic acid (EPA), and docosahexaenoic acid (DHA).

Exemplary hydrocarbon oils include squalane, liquid paraffin, polyisobutene, and vaseline.

Exemplary fluorochemical oils include perfluorodecalin, perfluorobutyltetrahydrofuran, perfluoroalkane, perfluoropolyether, and fluorine-modified silicone.

As the oily ingredient, the cosmetic composition can contain an organic UV absorbent. Preferred are oil-soluble or oleophilic liquid substances. Exemplary ones include 4-(1,1-dimethylethyl)-4'-methoxybenzoylmethane (trade name: Parsol 1789), octyl methoxycinnamate (trade name: Parsol MCX), methylbis(trimethylsiloxy)silylisopentyl trimethoxycinnamate (trade name: Sunshelter SP), 2-ethylhexyl paradimethylamino-benzoate (trade name: Escalol 507D), methyl paradimethylamino-benzoate (trade name: Escalol 506), 2,4-bis-[{4-(2-ethylhexoloxy)-2-hydroxy}-phenyl]-6-(4-methoxyph enyl)-1,3,5-triazine (trade name: Tinosorb S), octyltriazone (trade name: Uvinul T150), and octocrylene or 2-ethylhexyl 2-cyano-3,3-diphenylacrylate (trade name: Palsol 340).

Exemplary higher alcohols include linear alcohols, such as lauryl alcohol, cetyl alcohol, stearyl alcohol, behenyl alcohol, myristyl alcohol, oleyl alcohol, and cetostearyl alcohol; branched alcohols, such as monostearylglycerol ether (batyl alcohol), 2-decyltetradecynol, lanolin alcohol, cholesterol, phytosterol, hexyldodecanol, isostearyl alcohol, and octyldodecanol.

Exemplary ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentyl glycol dicaprylate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, tri-2-heptylundecanoic glyceride, castor oil fatty acid methyl ester, oleyl oleate, acetoglyceride, 2-heptylundecyl palmitate, diisobutyl adipate, 2-octyldodecyl n-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, other dimer acid esters than di (isostearyl/phytosteryl) dimer dilinoleate [for example, di(phytosteryl/behenyl) dimer dilinoleate, and (phytosteryl/isostearyl/cetyl/stearyl/behenyl) dimer dilinoleate)], pentaerythrityl tetra(behenate/benzoate/ethylhexanoate), pentaerythrityl tetra(ethylhexanoate/benzoate), and macadamia seed oil polyglyceryl-6-esters behenate.

Exemplary silicone oils include linear polysiloxanes, such as dimethylpolysiloxane, methylphenylpolysiloxane, and diphenylpolysiloxane; cyclic polysiloxanes, such as octamethylcyclotetrasiloxane, decamethylcyclopentasiloxane, and dodecame-thylcyclohexasiloxane; silicone resins forming three-dimensional networks; silicone rubbers; various modified polysiloxanes, such as amino-modified polysiloxanes, polyether-modified polysiloxanes, alkyl-modified polysiloxanes, and fluorine-modified polysiloxanes.

The others that do not basically dissolve in water are dealt with as oily ingredients in the invention, and exemplary ones include oil-soluble surfactants, oil-soluble chemicals, and other oil-soluble preservatives and antioxidants.

The total amount of the oily ingredients in the invention is from 1 to 20% by mass based on the total amount of the cosmetic composition (final product), more preferably from 7 to 20% by mass. When the total amount of the oily ingredients is more than 20% by mass, there may occur some inconvenience such that the adherability of the pressed powder composition to a cosmetic puff or sponge may become worsen.

### <Powder Ingredient>

In the pressed powder cosmetic composition of the invention, the powder ingredient may be any one generally usable in cosmetic compositions, and is not specifically defined. In concrete terms, exemplary ones include pigments (body pigments, color pigments, etc.), such as titanium dioxide, zinc oxide, red iron oxide, yellow iron oxide, black iron oxide, ultramarine, prussian blue, cerium oxide, talc, mica, sericite, kaolin, silica, zinc stearate, fluorine-containing phlogopite, synthetic talc, barium sulfate, boron nitride, bismuth oxychloride, alumina, and magnesium carbonate; polymer powders, such as silicone powder, silicone elastic powder [e.g., Torayfil E-506C (by Dow Corning Toray Co., Ltd.)], polyurethane powder, cellulose powder, nylon powder, PMMA powder, starch, polyethylene powder, and fluororesin powder [e.g., Teflon™ powder]; functional powders, such as zinc oxide fine powders and titanium oxide fine powders serving as a UV protective agent, sebum-adsorbing hydroxyapatite and its composite powder, porous polymer powder, and fibrous powder; but not limited thereto.

Of those, boron nitride excellent both in the impact resistance and the feeling in use of the cosmetic composition is preferably incorporated as a body pigment. The amount of boron nitride is preferably from 5 to 60% by mass, more preferably from 10 to 40% by mass.

### [Spherical Powder]

For improving the feeling in use of the cosmetic composition, a spherical powder may be incorporated in the composition as the powder ingredient. Exemplary spherical powder include spherical polyethylene powder, organopolysiloxane elastomer spherical powder or composite spherical powder containing it as the mother powder, polyurethane spherical powder or composite spherical powder containing it as the mother powder. As commercial products, exemplary ones include Torayfil E-505C, Torayfil E-506C, Torayfil E-506S, DC EP-9261TI COSMETIC POWDER, DC EP-9289LL COSMETIC POWDER, DC EP-9293AI COSMETIC POWDER, DC 9701 COSMETIC POWDER (by Dow Corning Toray Silicone Co., Ltd.), Silicone Powder KSP-100 Silicone Powder KSP-300 (by Shin-Etsu Chemical Co., Ltd.), Plastic Powder D-400, Plastic Powder D-800 (by Toshiki Pigment Co., Ltd.).

It is known that the incorporation of a spherical powder may worsen the impact resistance; however, even when a spherical powder is incorporated in the cosmetic composition of the invention, the impact resistance thereof is still excellent. The amount of the spherical powder is generally from 1 to 10% by mass, preferably from 1 to 5% by mass. The mean particle size of the spherical powder is preferably from 1 to 30 µm or so.

### [Pearlescent Pigment]

One of the characteristic features of the invention is that, even when a large pearlescent pigment having a weight-average particle size of 15 µm or more, especially 30 µm or more is incorporated in a large amount therein, the cosmetic composition still secures high impact resistance. Heretofore, in a pressed powder cosmetic composition containing a large amount of pearlescent pigments therein, it has been problematic in its poor impact resistance due to the powdery characteristics of the pearlescent pigment therein; however, since the specific dimer acid ester is used in the invention, the cosmetic composition still secures high impact resistance even when a pearlescent pigment is incorporated in a large amount therein.

The above described advantageous effects are noticeable when the incorporation of the pearlescent pigment in a ratio of from 10 to 70% by mass, especially from 15 to 50% by mass, based on the total amount of the cosmetic composition.

The pearlescent pigment is a tabular powder having a pearlescent gloss, and is a general purpose use powder in the field of coating materials and cosmetic materials. Exemplary ones include mica titanium, iron oxide-coated mica titanium, carmine-coated mica titanium, carmine/prussian blue-coated mica titanium, iron oxide/carmine-treated mica titanium, prussian blue-treated mica titanium, iron oxide/prussian blue-treated mica titanium, chromium oxide-treated mica titanium, black titanium oxide-treated mica titanium, acrylic resin-coated aluminium powder, silica-coated aluminium powder, titanium oxide-coated mica, titanium oxide-coated bismuth oxychloride, titanium oxide-coated talc, colored titanium oxide-coated mica, titanium oxide-coated synthetic mica, titanium oxide-coated silica, titanium oxide-coated alumina, titanium oxide-coated glass powder, polyethylene terephthalate/polymethyl methacrylate laminate film powder, bismuth oxychloride, and argentine.

As commercial products, exemplary ones include Timiron Splendid Gold™, Timiron Splendid Red™, Timiron Splendid Blue™, Timiron Splendid Green™, Timiron Super Red™, Timiron Super Blue™, Timiron Super Green™, Timiron Super Gold™, Colorona Sienna™, Colorona Carmine Red™, Colorona Red Gold™ (all by Merck & Co., Inc.), Cloisonne Blue™, Cloisonne Green™, Cloisonne Gold™, Cloisonne Rouge Flambe™, Gemtone Tan Opal™, Gemtone Ruby™, Timica Brilliant Gold™, Timica Golden Bronze™, Timica Copper™, Duochrome RB™, Duochrome RY™, Duochrome YR™, Duochrome YB™, Duochrome RG™, Duochrome BG™, Duochrome BR™, Duochrome GY™, Flamenco Velvet™, Flamenco Satina™, Flamenco Red™, Flamenco Blue™, Flamenco Gold™ (all by BASF), and Metashine™ Series (all by NSG Group).

The above-mentioned powder ingredients for use herein may be those of which the surface is untreated, or may also be those surface-treated with any of silicone or fluorine compounds, or silane coupling agents, fluororesins [e.g., Teflon™], fatty acids, fatty acid soaps, lauroyl lysine or the like. One or more of the powder ingredients may be used here either singly or as combined.

The total amount of the powder ingredients in the invention is from 80 to 99% by mass, based on the total amount of the cosmetic composition (final product), more preferably from 80 to 93% by mass.

### <Other Optional Ingredients>

In addition to the above-mentioned ingredients, any other ingredient generally usable in skincare external preparations such as cosmetic compositions and medicinal products or the like may be suitably incorporated in the pressed powder cosmetic composition of the invention, as long as not detracting from the advantageous effects of the invention; and those ones include anionic surfactants, cationic surfactants, ampholytic surfactants, nonionic surfactants, moisturizers, water-soluble polymers, thickeners, film-forming agents, UV absorbents, sequestering agents, lower alcohols, polyalcohols, saccharides, amino acids, organic amines, polymer emulsions, pH-controlling agents, skincare nutrients, vitamins, antioxidants, antioxidation promoters, fragrances, water, preservatives and the like.

### <Method for Producing Pressed Powder Cosmetic Composition>

Production of the pressed powder cosmetic composition of the invention is not specifically defined. According to a "dry molding process" of mixing and molding the powder ingredient and the oil ingredient without using a solvent, the intended advantageous effects could be attained; however, a "wet molding process" described in detail hereinunder is preferred here.

By the "wet molding process", the cosmetic composition is, for example, produced by as follows. First, powder ingredients are mixed in a Henschel mixer or the like, then oily ingredients including the dimer acid ester are added thereto and uniformly mixed to prepare a cosmetic base. Next, the cosmetic base is mixed with a solvent to give a slurry. Thusly obtained slurry is filled in a container. While filling, in the case where the slurry poorly spreads in the container, the container may be lightly shaken to such an extent that the filled slurry does not run over from the container, whereby the slurry could be filled uniformly. After being filled in the container, the solvent is removed from the slurry for solidification. The removal of the solvent may be conducted in an ordinary manner by spontaneous drying, heating drying, hot air drying, vacuum suction or the like. The above-mentioned production method is a so-called wet molding process, and its details are described in Patent References 3 and 4 mentioned hereinabove in "PRIOR ART REFERENCES".

The amount of the solvent may vary depending on, for instance, the formulation of the powder ingredients and the amount of the oily ingredients, but is preferably so controlled that the viscosity of the slurry could facilitate the removal of air from it and that the slurry could be easily filled into the container. In general, the amount of the solvent is preferably from 0.5 to 1.5 times (by weight) the amount of the cosmetic base. When the solvent amount is too large, then a long time must be taken for drying, and in addition, after dried, the solidified composition may be cracked or the weight thereof may reduce, and the impact resistance thereof may also be lowered.

As the solvent for use in the invention, it includes water, alcohols (e.g., methanol, ethanol, and isopropyl alcohol), benzene, toluene, THF, paraffin, and silicone. One or more such solvents may be used either singly or as combined, depending on the characteristics of the powder ingredients and the oily ingredients to be used. Of those, preferred is ethanol.

When the cosmetic composition is produced according to the wet molding process, the oily ingredients and others may be removed away along with the solvent. Therefore, it is necessary to increase the blending quantities in the production, relative to the intended contents in the final product. The degree of increasing the quantities may vary depending on the types and the amounts of the blending ingredients and the solvent, the production condition and others, and therefore may be suitably determined. In many cases, it is desirable that the quantity increase is from 120 to 150% by mass; however, in the case where the compatibility of the constitutive ingredients with the solvent is high, the quantity increase will have to be 200% by mass or so.

The pressed powder cosmetic composition of the invention can be used, for example, as a makeup cosmetic composition, such as eye shadow, face powder, blusher (cheek color), foundation or the like. In particular, the invention exhibits an especially excellent advantageous effects in eye shadow and blusher in which a large amount of a pearlescent pigment may be incorporated.

### EXAMPLES

Examples of the invention are given below, by which, however, the invention should not be limited. Unless otherwise specifically indicated, the amount is in terms of % by mass, based on the total amount of the final product of the cosmetic composition.

### <Evaluation Criteria>

First described are the evaluation criteria used in the invention.

### [Impact Resistance]

A molded product of the cosmetic composition was set in a compact container, and while cased it in a box with the compact container facing downward, and it was dropped down onto a metal plate from a height of 30 cm. The above-noted dropping test was repeated until the cracks first appear in the cosmetic composition, and counted as to how many times the dropping test was conducted till the first appearance of the cracks (N = 3). Following each test, the impact resistance of the product was evaluated by the averaged number of dropping test times. The number of dropping test was determined to be at most 50 times.

### [Light Spreadability]

Twelve women expert panelists applied each cosmetic composition onto their skin and evaluated the light spreadability thereof on their skin in 5 ranks (5: very good, 4: good, 3: average, 2: bad, 1: very bad). By the averaged evaluation, the light spreadability of the cosmetic composition was evaluated as follows:
Θ: 4 or more.
○: From 3 to less than 4.
Δ: From 2 to less than 3.
x: Less than 2.

### [Adherability]

Twelve women expert panelists tried to take the cosmetic composition with a powder puff and evaluated for the adherability thereof to the puff in 5 ranks (5: very good, 4: good, 3: average, 2: bad, 1: very bad). By the averaged evaluation, the adherability of the cosmetic composition was evaluated as follows:
Θ: 4 or more.
○: From 3 to less than 4.
Δ: From 2 to less than 3.
x: Less than 2.

### [Color Expressibility]

Twelve women expert panelists applied each cosmetic composition onto their skin and evaluated the color expressibility thereof on their skin in 5 ranks (5: very good, 4: good, 3: average, 2: bad, 1: very bad). By the averaged evaluation, the color expressibility of the cosmetic composition was evaluated as follows:
Θ: 4 or more.
○: From 3 to less than 4.
Δ: From 2 to less than 3.
x: Less than 2.

### <Production Method>

The powder ingredients were mixed in a Henschel mixer or the like, then the oily ingredients were added thereto and mixed uniformly to prepare a cosmetic base. Ethanol was added thereto in an amount of from 60 to 80% by mass, and uniformly mixed to give a slurry. As a part of the oily ingredients were to be evaporated away in the following step of removing ethanol, the oily ingredients were incorporated in an increased quantity of from 120 to 160% so that the blending quantities in the final product could be the prescribed quantities. The slurry was filled in a medium-size plate and compression-molded using a molding head (compression pressure, 20 kg), and at the same time, ethanol was sucked away through the back of the molding head. After the suction, the molded product was dried at 50°C for 2 hours.

### (Test Example 1)

A pressed powder cosmetic was prepared according to the prescription shown in Table 1, and this was tested and evaluated for the impact resistance and the feeling in use, etc., according to the above-mentioned test methods. The results are shown in Table 1.

In Test Examples 1-2 to 1-8, used was a semisolid or highly-viscous ester oil similar to the specific dimer acid ester that is the indispensable ingredient in the invention in point of the structure and the physical properties thereof, or vaseline popularly used in pressed powder cosmetic compositions produced in a wet molding process, for the purpose of comparing it with the specific dimer acid ester. These ester oils are all characterized by water absorbability and glossiness with high refractivity, and are used in oily cosmetic compositions such as rouge, etc.

In Table 1, the following compounds were used the commercial products below.

Di(isostearyl/phytosteryl) dimer dilinoleate^{(*1)}: LUSPLAN PI-DA (by Nippon Fine Chemical Co., Ltd.).
Macadamia seed oil polyglyceryl-6-esters behenate^{(*2)}: S-FACE VL-211 (by Sakamoto Yakuhin Kogyo Co., Ltd.).
(Phytosteryl/isostearyl/cetyl/stearyl/behenyl) dimer dilinoleate^{(*3)}: Plandool S (by Nippon Fine Chemical Co., Ltd.).
(Phytosteryl/isostearyl/cetyl/stearyl/behenyl) dimer dilinoleate^{(*4)}: Plandool H (by Nippon Fine Chemical Co., Ltd.).

**[Table 1]**

| | Test Ex.1-1 | Test Ex.1-2 | Test Ex.1-3 | Test Ex.1-4 | Test Ex.1-5 | Test Ex.1-6 | Test Ex.1-7 | Test Ex.1-8 |
|---|---|---|---|---|---|---|---|---|
| Silicone-Treated Talc | bal. | bal. | bal. | bal. | bal. | bal. | bal. | bal. |
| Synthetic Mica | 30 | 30 | 30 | 30 | 30 | 30 | 30 | 30 |
| Boron Nitride | 10 | 10 | 10 | 10 | 10 | 10 | 10 | 10 |
| Pearlescent Pigment | 15 | 15 | 15 | 15 | 15 | 15 | 15 | 15 |
| Spherical Polyethylene Powder | 1 | 1 | 1 | 1 | 1 | 1 | 1 | 1 |
| Silicone-Treated Red Iron Oxide | 3 | 3 | 3 | 3 | 3 | 3 | 3 | 3 |
| Silicone-Treated Yellow Iron Oxide | 3 | 3 | 3 | 3 | 3 | 3 | 3 | 3 |
| Silicone-Treated Black Iron Oxide | 2.1 | 2.1 | 2.1 | 2.1 | 2.1 | 2.1 | 2.1 | 2.1 |
| Difisostearyl/phytosteryl) Dimer Dilinoleate^{(*1)} | 3 | - | - | - | - | - | - | - |
| Pentaerythrityl Tetra(behenate/benzoate/athylhaxanoate) | - | 3 | - | - | - | - | - | - |
| Pentaerythrityl Tatra(ethylhexanoate/benzoate) | - | - | 3 | - | - | - | - | - |
| Macadamia Seed Oil Polyglyceryt-6-Esters Behanate^{(*2)} | - | - | - | 3 | - | - | - | - |
| Di(phytosteryl/behenyl) Dimer Dilinoleate | - | - | - | - | 3 | - | - | - |
| (Phytosteryl/isostearyl/cetyl/stearyl/behenyl) imer Dilinoleate^{(*3)} | - | - | - | - | - | 3 | - | - |
| (Phytosteryllisostearyl/oetyl/slearyllbehenyl) Dimer Dilinoleate^{(*4)} | - | - | - | - | - | - | 3 | - |
| Vaseline | - | - | - | - | - | - | - | 3 |
| Dimethylpolysiloxane | 0.5 | 0.5 | 0.5 | 0.5 | 0.5 | 0.5 | 0.5 | 0.5 |
| Glyceryl Tri-2-athylhexanoate | 2 | 2 | 2 | 2 | 2 | 2 | 2 | 2 |
| Sorbitan Sesqui-isostearate | 1 | 1 | 1 | 1 | 1 | 1 | 1 | 1 |
| Chlorphenesin | 0.2 | 0.2 | 0.2 | 0.2 | 0.2 | 0.2 | 0.2 | 0.2 |
| Tocopherol | 0.02 | 0.02 | 0.02 | 0.02 | 0.02 | 0.02 | 0.02 | 0.02 |
| Impact Resistance (dropping test, number of dropping times) | 41.7 | 17.3 | 15 | 18.3 | 21.3 | 18 | 18 | 11.7 |
| Light Spreadability | Θ | × | × | Θ | ○ | Δ | Δ | Θ |
| Adherability | ○ | Δ | Δ | ○ | ○ | ○ | ○ | ○ |
| Color Expressibility | Θ | ○ | ○ | Θ | ○ | ○ | ○ | Θ |

As obvious from the results in Table 1, it has been confirmed that when di(isostearyl/phytosteryl) dimer dilinoleate is incorporated therein, a pressed powder cosmetic composition being excellent in impact resistance and having light spreadability can be obtained (Test Example 1-1).

As opposed to this, the samples with a different ester oil incorporated therein (Test Example 1-2 to Test Example 1-7) were improved in some degree in point of the impact resistance as compared with the sample with a general-purpose oily ingredient, vaseline incorporated therein (Test Example 1-8); however, the impact resistance of those samples could not be so remarkably improved like that of the sample of the invention with di(isostearyl/phytosteryl) dimer dilinoleate incorporated therein.

As in the above, it is known that, when di(isostearyl/phytosteryl) dimer dilinoleate is incorporated in a pressed powder cosmetic composition, then the impact resistance of the cosmetic composition can be remarkably improved to such an extent that it could not be anticipated from the results of the other samples with any other similar ingredient incorporated therein.

In addition, it is also known that the incorporation of di(isostearyl/phytosteryl) dimer dilinoleate (3% by weight in Table 1) provides a specific pressed powder cosmetic composition satisfying both impact resistance and good feeling in use.

### (Test Example 2)

Next, the blending quantity of di(isostearyl/phytosteryl) dimer dilinoleate in the pressed powder cosmetic composition of the invention was investigated, and the results are shown in Table 2. The production method is the same as in Test Example 1. Di(isostearyl/phytosteryl) dimer dilinoleate is the same commercial product as used in Test Example 1.

**[Table 2]**

| | Test Ex.2-1 | Test Ex.2-2 | Test Ex.2-3 | Test Ex.2-4 | Test Ex.2-5 | Test Ex.2-6 | Test Ex.2-7 | Test Ex.2.8 | Test Ex.2-9 | Test Ex.2-10 |
|---|---|---|---|---|---|---|---|---|---|---|
| Silicone-Treated Talc | bal. | bal. | bal. | bal. | bal. | bal. | bal. | bal. | bal. | bal. |
| Synthetic Mica | 10 | 10 | 10 | 10 | 10 | 10 | 10 | 10 | 10 | 10 |
| Boron Nitride | 10 | 10 | 10 | 10 | 10 | 10 | 10 | 10 | 10 | 10 |
| Pearlescent Pigment | 35 | 35 | 35 | 35 | 35 | 35 | 35 | 35 | 35 | 35 |
| Spherical Polyethylene Powder | 1 | 1 | 1 | 1 | 1 | 1 | 1 | 1 | 1 | 1 |
| Silicone-Treated Red Iron Oxide | 3 | 3 | 3 | 3 | 3 | 3 | 3 | 3 | 3 | 3 |
| Silicone-Treated Yellow Iron Oxide | 3 | 3 | 3 | 3 | 3 | 3 | 3 | 3 | 3 | 3 |
| Silicone-Treated Black Iron Oxide | 2.1 | 2.1 | 2.1 | 2.1 | 2.1 | 2.1 | 2.1 | 2.1 | 2.1 | 2.1 |
| Di(isostearyl/phytosteryl) Dimer Dilinoleate^{(*1)} | 0.5 | 1 | 3 | 10 | 15 | 20 | - | - | - | - |
| Vaseline | - | - | - | - | - | - | - | 0.5 | 1 | 3 |
| Dimethylpolysiloxane | 0.5 | 0.5 | 0.5 | 0.5 | 0.5 | 0.5 | 0.5 | 0.5 | 0.5 | 0.5 |
| Glyceryl Tri-2-ethylhexanoate | 4.5 | 4 | 2 | - | - | - | 5 | 4.5 | 4 | 2 |
| SorbitanSesqui-isostearate | 1 | 1 | 1 | 1 | 1 | 1 | 1 | 1 | 1 | 1 |
| Worphenesin | 0.2 | 0.2 | 0.2 | 0.2 | 0.2 | 0.2 | 0.2 | 0.2 | 0.2 | 0.2 |
| Tocopherol | 0.02 | 0.02 | 0.02 | 0.02 | 0.02 | 0.02 | 0.02 | 0.02 | 0.02 | 0.02 |
| Impact Resistance (dropping test, number of dropping times) | 6.7 | 11.7 | 18.3 | 50< | 50< | 50< | 5 | 5.3 | 4.7 | 9.7 |
| Spreadability | Θ | Θ | Θ | ○ | ○ | Δ | Θ | Θ | Θ | Θ |
| Adherability | Δ | ○ | Θ | Θ | Δ | × | × | Δ | Δ | ○ |
| Color Expressibility | ○ | Θ | Θ | ○ | ○ | ○ | × | Δ | ○ | ○ |

As obvious from the results in Table 2, even when a small amount, 0.5% by mass of di(isostearyl/phytosteryl) dimer dilinoleate is incorporated, it is effective for improving the impact resistance of the cosmetic composition, as compared with the other oily ingredients (Test Example 2-1, Test Example 2-8). In addition, it has been confirmed that 1% by mass of the ingredient is effective for improving the impact resistance (Test Example 2-2). It is known that, when the blending quantity of di(isostearyl/phytosteryl) dimer dilinoleate is from 0.5 to 15% by mass, especially from 1 to 10% by mass, the balance of the impact resistance and the feeling in use of the cosmetic composition is extremely excellent (Test Example 2-2 to Test Example 2-5). On the other hand, when the ingredient was incorporated in an amount of 20% by mass, the total amount of the oily ingredients was 21.5% by mass and was large, and therefore the adherability of the cosmetic composition was worsened (Test Example 2-6).

Preferred Formulation Examples of the pressed powder cosmetic composition of the invention are described below. For these, the production method is the same as that in the above-mentioned Test Examples 1 and 2.

### (Formulation Example 1) Cheek Color

| (Constitutive Ingredients) | (% by mass) |
|---|---|
| Di(isostearyl/phytosteryl) Dimer Dilinoleate | 3 |
| Vaseline | 2 |
| Glyceryl tri-2-ethylhexanoate | 4 |
| Methylpolysiloxane | 6 |
| Sorbitan Sesqui-isostearate | 1 |
| Pearlescent Pigment (trade name: Timiron MP-115) | 10 |
| Mica | 40 |
| Red Iron Oxide | 2 |
| Talc | bal. |

### (Formulation Example 2) Eye Shadow

| (Constitutive Ingredients) (% | by mass) |
|---|---|
| Di(isostearyl/phytosteryl) Dimer Dilinoleate | 5 |
| Glycerol Tri-2-ethylhexanoate | 3 |
| Methylpolysiloxane | 3 |
| Sorbitan Sesqui-isostearate | 5 |
| Pearlescent Pigment (trade name: Timiron MP-115) | 20 |
| Mica | 20 |
| Silicone-treated Red Iron Oxide | 2 |
| Talc | bal. |

### (Formulation Example 3) Foundation

| (Constitutive Ingredients) | (% by mass) |
|---|---|
| Di(isostearyl/phytosteryl) Dimer Dilinoleate | 3 |
| Vaseline | 1 |
| Glyceryl tri-2-ethylhexanoate | 4 |
| Methylpolysiloxane | 1 |
| Sorbitan Sesqui-isostearate | 1 |
| Mica | 20 |
| Red Iron Oxide | 2 |
| Yellow Iron Oxide | 2 |
| Black Iron Oxide | 1 |
| Boron Nitride | 20 |
| Talc | bal. |

### (Formulation Example 4) Dual Foundation

| (Constitutive Ingredients) | (% by mass) |
|---|---|
| Di(isostearyl/phytosteryl) Dimer Dilinoleate | 4 |
| Glyceryl tri-2-ethylhexanoate | 1 |
| Methylpolysiloxane | 3 |
| Sorbitan Sesqui-isostearate | 1 |
| Silicone-treated Mica | 20 |
| Silicone-treated Red Iron Oxide | 2 |
| Silicone-treated Yellow Iron Oxide | 2 |
| Silicone-treated Black Iron Oxide | 1 |
| Silicone-treated Talc | bal. |

### (Formulation Example 5) Eye Shadow

| (Constitutive Ingredients) | (% by mass) |
|---|---|
| Di(isostearyl/phytosteryl) Dimer Dilinoleate | 2 |
| Macadamia Seed Oil Polyglyceryl-6-Esters Behenate | 2 |
| Dimethylpolysiloxane | 1 |
| Glycerol Tri-2-ethylhexanoate | 2 |
| Sorbitan Sesqui-isostearate | 1 |
| Synthetic Mica | 10 |
| Boron Nitride | 10 |
| Mg Myristate-treated Tabular Barium Sulfate | 10 |
| Pearlescent Pigment | 25 |
| Silicone-treated Red Iron Oxide | 3 |
| Silicone-treated Yellow Iron Oxide | 3 |
| Silicone-treated Black Iron Oxide | 2 |
| Chlorphenesin | 0.2 |
| Tocopherol | 0.02 |
| Silicone-treated Talc | bal. |

### (Formulation Example 6) Pressed Powder

| (Constitutive Ingredients) | (% by mass) |
|---|---|
| Di(isostearyl/phytosteryl) Dimer Dilinoleate | 2 |
| Vaseline | 1 |
| Glyceryl Tri-2-ethylhexanoate | 2 |
| Methylpolysiloxane | 1 |
| Sorbitan Sesqui-isostearate | 1 |
| Boron Nitride | 5 |
| Talc | bal. |

### INDUSTRIAL APPLICABILITY

According to the invention, there is provided a solid cosmetic excellent in impact resistance. According to the invention, there is also provided a pressed powder cosmetic composition satisfying both impact resistance and good feeling in use.

## Claims

1. A pressed powder cosmetic composition comprising oily ingredients in an amount of from 1 to 20% by mass and powder ingredients in an amount of from 80 to 99% by mass, wherein the oily ingredients include di(isostearyl/phytosteryl) dimer dilinoleate.

2. The pressed powder cosmetic composition as claimed in claim 1, wherein di (isostearyl/phytosteryl) dimer dilinoleate is contained in a ratio of from 0.5 to 15% by mass in the total amount of the cosmetic composition.

3. The pressed powder cosmetic composition as claimed in claim 1 or 2, wherein the powder ingredients include a pearlescent pigment in a ratio of from 10 to 70% by mass in the total amount of the cosmetic composition.

4. The pressed powder cosmetic composition as claimed in claim 1 or 2, wherein the powder ingredients further include boron nitride in an amount of from 5 to 60% by mass in the total amount of the cosmetic composition.

5. The pressed powder cosmetic composition as claimed in claim 1 or 2, which is obtainable by adding a solvent to a cosmetic base containing powder ingredients and oily ingredients, to thereby prepare a slurry, then filling the slurry in a container, and thereafter removing the solvent.
